Europäisches Patentamt

European Patent Office

Office européen des brevets

(19)

(11) **EP 0 585 234 B1**

(12) **EUROPEAN PATENT SPECIFICATION**

(45) Date of publication and mention
of the grant of the patent:
**27.11.1996 Bulletin 1996/48**

(21) Application number: **92905503.6**

(22) Date of filing: **27.02.1992**

(51) Int Cl.6: **C12N 1/00**, C12N 1/20

(86) International application number:
**PCT/DK92/00060**

(87) International publication number:
**WO 92/19716 (12.11.1992 Gazette 1992/28)**

(54) **METHOD FOR OBTAINING A CULTURE MEDIUM FROM PLANT SAP**

METHODE ZUR ERHALTUNG EINES KULTURMEDIUM AUS PFLANZENSAFT

PROCEDE D'OBTENTION DE MILIEUX DE CULTURE A PARTIR DE SEVES VEGETALES

(84) Designated Contracting States:
**DE ES FR GB IT NL SE**

(30) Priority: **07.05.1991 DK 839/91**

(43) Date of publication of application:
**09.03.1994 Bulletin 1994/10**

(73) Proprietor: **AGRO-FERM A/S**
**DK-6870 Olgod (DK)**

(72) Inventors:
• **KIEL, Pauli**
**DK-6731 Tj reborg (DK)**

• **ANDERSEN, Margrethe**
**DK-6731 Tj reborg (DK)**

(74) Representative: **Hansen, Kaj**
**c/o Kaj Hansen ApS,**
**Radgivende ingeniorfirma,**
**Elsegarde Skovvej 5**
**8400 Ebeltoft (DK)**

(56) References cited:
EP-A- 0 065 246          CH-A- 523 963
FR-A- 904 488            GB-A- 2 090 288

**Description**

The present invention relates to a procedure for conversion of plant juice to a medium which is well suited as a nutritive medium for micro-organisms requiring vitamin and amino acids, for example bacteria which form organic acids or amino acids.

In connection with agro-industrial productions, for example production of potato starch and vegetable pellets, there will be a residual product in the shape of a liquid or juice, which hitherto has been sprayed as manure on fields.

The juice has a content of dry matter of 2%-6%. This dry matter contains among other things fibres, protein, carbohydrates, vitamins and free amino acids.

This juice thus contains all the substances which are necessary to promote growth of the most demanding micro-organisms.

However, the juice can only be used as substrates for micro-organisms which do not make heavy demands on the content of free amino acids, or which are capable of forming proteolytic exoenzymes, e.g. bacillus species.

Thus, bacteria of the family Lactobacillus and auxotrophic mutants which have specific amino-acid demands, e. g. mutants of Corynebacterium glutamicum and Brevibacterium lactofermentum, which are used for the production of amino acids, e.g. glutamic acid, L-lysine and threonine, cannot spontaneously utilize the plant juice because most amino acids are bonded in the proteins. This fermentation process is slow and therefore not applicable in practice.

It is well known that it is possible to hydrolyse protein to free amino acids either by acid hydrolysis by using strong acids and high temperatures or by enzymic hydrolysis using protease and peptidase.

However, it is not possible to carry out acid hydrolysis on an aqueous solution of protein of so low a concentration as the liquid in question.

Nor will it be possible to use enzymic hydrolysis, as it will be necessary to use very large quantities of enzymes so that the process will be too expensive and therefore economically forbidding.

It is the purpose of the present invention to describe a procedure by which the plant juice can be converted into a medium which is well suited as a nutritive substrate to culture e.g. Lactobacillus delbruckii, Lactobacillus plantarum and amino-acid demanding mutants of Brevibacterium lactofermentum and Corynebacterium glutamicum.

This procedure makes it possible to utilize the plant juice in an industrial fermentation process in the production of e.g. lactic acid or amino acids, which both are products which find extensive application in the food and feedstuffs industries and the chemical industry.

This is achieved by the described procedure in the characterizing part of claim 1.

By the said procedure it is also possible as described in claim 2 to use enzymes occurring naturally in some plant juices.

Claim 3 deals with a procedure for production of enzymes to be used in the hydrolysis, and

Claim 4 deals with enzymic treatment of the plant juice with commercial enzymes, e.g. ALCALASE[R].

The following examples serve to illustrate the invention.

**EXAMPLE 1**

In the production of vegetable pellets the cut grass or alfalfa is pressed at once, thereby producing vegetable juice, or it is heated first and then pressed, whereby brown juice is produced.

The brown juice contains 2-5% dry matter of which 15-35% is protein, abt. 22% ashes, and 20-40% is carbohydrate.

In this case a vacuum evaporated brown juice concentrate has been produced.

This concentrated brown juice contains 22% dry matter of which 5% is protein.

The concentrated brown juice is heated to 80°C for ten hours in order to pasteurize the brown juice.

The temperature is set at 55°C, pH is set at 8.0 with 6N ammonia, whereafter the temperature is held at 55°C and the pH at 8.0 (pH-Stat) with current adjustment of the pH by addition of 6N ammonia.

The hydrolysation is stopped when the consumption of base corrssponds to a degree of hydrolysis (DH) of a minimum of 80%.

The degree of hydrolysis is calculated as follows:

$$DH = B \cdot \frac{1}{\alpha} \cdot \frac{N_B}{MP} \cdot \frac{1}{h_{tot}} \cdot 100\%, \text{ where}$$

B:      Consumption of base in litres.

$\frac{1}{\alpha}$       Calibration factor for pH-Stat, at pH=8 is used the value 1.0.

$N_B$:       Normality of base.

MP:       Protein mass in kg.

$h_{tot}$:       Total number of peptide bonds. Here $h_{tot}$=8.0 is used.

In this example 122 ml 6N ammonia is consumed after 24 hours for hydrolysis of 2 1 concentrated brown juice, corrdesponding to a degree of hydrolysis of 91.5%.

The hydrolysis process is stopped.

The hydrolyzed concentrated brown juice is now used in a culture medium to culture Corynebacterium glutamicum ATCC 21526, a strain demanding homoserine and leucine.

The culture medium is composed as follows:

10% molassis (calculated as glucose).

20% concentrated hydrolyzed brown juice, corresponding to 1% protein hydrolysate.

0.07% $KH_2PO_4$

0.05% $MgSO_4 . 7H_2O$

0.3% urea

0.5% $(NH_4)_2SO_4$

3% $CaCo_3$

pH set at 7.5.

Culture is carried out at 30°C for 4 x 24 hours in a 1-litre fermentor with aeration.

Culture in this nutritive medium produces 22.5 mg/ml of L-lysine.

Culture in a substrate with non-hydrolyzed brown juice produces only 4.3 mg/ml of L-lysine.

In a medium where the brown juice is substituted by soya bean cake sulphuric acid decomposate there is a production of 19.5 mg/ml of L-lysine.

## EXAMPLE 2

Potato fruit juice is one of the residual products in the production of starch.

Potato fruit juice contains the parts of the potato which in the process of leaching out of the starch have decomposed, including part of the nitrogenous compounds, protein, amino acids, etc.

The potato fruit juice used in the example contains 4.4% dry matter of which 2.0% is protein (N x 6.25).

The potato fruit juice is heat treated at 120°C for 20 min.

The heat treated potato fruit juice is cooled to 55°C, pH is set at 8.0 with 4N ammonia, whereafter 0.01% ALCALASE[R] 2.4L is added.

pH is kept constant at 8.0 (pH-Stat) by adding 4N ammonia or 4N sodium hydroxide.

After 4 hours a degree of hydrolysis (DH) of 35% is obtained.

The hydrolyzed potato fruit juice is ultrafiltrated so that particles and molecules with a molecular weight higher than 10,000 are removed.

The ultrafiltrate contains partially hydrolyzed protein and free amino acids.

The ultrafiltrate is added 2% glucose, pH is set at 7.0, it is autoclaved at 110°C for 15 min. and used as a culture medium for various bacteria. After incubation at 37°C for 24 hours the following growth is obtained, measured as optical density at 450nm ($OD_{450}$).

In the case of culture of potato fruit juice ultrafiltrate which is not subjected to enzymic treatment the growth will in most cases be smaller.

TABLE:

| Bacteria | Enzyme treated | Not enzyme treated |
|---|---|---|
| Corynebacterium glutamicum ATCC 21526 | 6.6 | 0.5 |
| Brevibacterium lactofermentum ATCC 21798 | 4.4 | 1.2 |
| Brevibacterium lactofermentum ATCC 13869 | 6.5 | 1.8 |
| Bacillus subtilis ATCC 6051 | 1.9 | 2.0 |
| Lactobacillus casei ATCC 11443 | 2.5 | 0.5 |
| Lactobacillus bulgaricus Chr.Hansen's Lab | 1.5 | 0.3 |

**EXAMPLE 3**

In the culture of Bacillus amyloliquefacieus ATCC 23842 in a sterile medium consisting of brown juice with a content of protein of 1% it is possible to produce proteolytic enzymes which can be used in a subsequent hydrolysis of plant juice.

After culture at 37°C for 24 hours under aeration an enzyme activity is obtained in the brown juice of $2.5 \times 10^{-2}$ Anson units/ml, which corresponds to the enzyme activity used when using commercial enzymes such as e.g. ALCALASE$^R$ 0.6L.

**Claims**

1.  Procedure for the conversion of plant juice to a medium which is well suited as a nutritive substrate for bacteria demanding vitamins and amino acids, which form organic acids or amino acids, **characterized** by the plant juice first being heat treated in an interval for related values of temperature and time, ranging from 55°C and 24 hours to 120°C and 10 minutes, depending on the nature of the plant juice, and by the subsequent cooling of the plant juice to 50°C - 60°C, and by the pH value being set at 7.5 - 8.5 by addition of a base, preferably ammonia, whereafter the plant juice is converted by enzymic hydrolysis by means of proteolytic enzymes, preferably proteases and peptidases under continuous agitation and by the pH value being kept constant at 7.5-8.5 under continued addition of a base until the hydrolysis process is over.

2.  Procedure for the conversion of plant juice according to claim 1, **characterized** by the fact that the enzymes are proteolytic enzymes occurring naturally in some plant juices, e.g. in grass juice.

3.  Procedure for the conversion of plant juice according to claim 1, **characterized** by the fact that the enzymes are proteolytic enzymes formed by micro-organisms cultivated in the plant juice or formed by the cultivation of micro-organisms in a nutritive substrate of a kind other than the plant juice which is to be converted.

4.  Procedure for the conversion of plant juice according to claim 1, **characterized** by the fact that the plant juice is hydrolyzed by addition of a proteolytic enzyme, e.g. ALCALASE®.

**Patentansprüche**

1.  Methode zur Umbildung von Pflanzensaft in ein Medium, das sich als Nährboden für vitamin- und aminosäurebedürftige Bakterien eignet, die organische Säuren oder Aminosäuren bilden, dadurch **gekennzeichnet**, dass der Pflanzensaft zunächst wärmebehandelt wird in einem Intervall für zusammengehörende Werte von Temperatur und Zeit von 55°C und 24 Std. bis 120°C und 10 Min., abhängig von der Art des Pflanzensafts, dass der Pflanzensaft danach auf 50°C - 60°C abgekühlt wird, und dass der pH-Wert auf 7,5 - 8,5 eingestellt wird unter Zufuhr einer Base, vorzüglich Ammoniak, wonach der Pflanzensaft mittels enzymatischer Hydrolyse und mittels proteolytischer Enzyme, vorzüglich Proteasen und Peptidasen, umgebildet wird, unter Umrühren und indem der pH-Wert auf 7,5-8,5 festgehalten wird bei weiterer Zufuhr von Base bis die Hydrolyse beendet ist.

2.  Methode zur Umbildung von Pflanzensaft dem Anspruch 1 entsprechend, dadurch **gekennzeichnet**, dass die Enzyme aus proteolytischen Enzymen bestehen, die natürlich in einigen Pflanzensaften, z.B. Grassaft, vorkommen.

3. Methode zur Umbildung von Pflanzensaft dem Anspruch 1 entsprechend, dadurch **gekennzeichnet**, dass die Enzyme aus proteolytischen Enzymen bestehen, die aus Mikroorganismen gebildet werden, die im Pflanzensaft gezüchtigt werden, oder durch Züchtigung von Mikroorganismen in einem Nährboden anderer Art als der Pflanzensaft, der umgebildet werden soll, gebildet sind.

4. Methode zur Umbildung von Pflanzensaft dem Anspruch 1 entsprechend, dadurch **gekennzeichnet**, dass der Pflanzensaft durch Hinzufügung eines proteolytischen Enzyms, z.B. ALCALASE® hydrolisiert wird.

**Revendications**

1. Procédé pour la transformation de la sève en un agent apte à utiliser comme substrat nutritif pour les microorganismes ayant besoin de vitamines et d'acide aminé, par exemple les bactéries qui produisent des acides organiques ou des acides aminés, **caractérisé** en ce que la sève est d'abord traitée thermiquement pendant un intervalle pour valeurs connexes de température et de temps allant de 55°C et 24 heures à 120°C et 10 min. selon la nature de la sève, en ce que la température de la sève est ensuite réduite à 50°C - 60°C, et en ce que la valeur pH est réglée à 7,5 - 8,5 en ajoutant une base, préférablement de l'ammoniac, après quoi la sève est transformée par hydrolyse enzymatique à l'aide d'enzymes protéolytiques, préférablement des protéases et des peptidases en brassant et en maintenant une valeur pH constante de 7,5 - 8,5 par l'addition continue de base jusqu'à la fin de l'hydrolyse.

2. Procédé pour la transformation de la sève selon la revendication 1, **caractérisé** en ce que les enzymes sont des enzymes protéolytiques qui se rencontrent naturellement dans quelques sèves tel que le jus de graminée.

3. Procédé pour la transformation de la sève selon la revendication 1, **caractérisé** en ce que les enzymes sont des enzymes protéolytiques produites par des microorganismes qui sont cultivés dans la sève, ou qui ont été produites par la culture de microorganismes dans un substrat nutritif autre que la sève à transformer.

4. Procédé pour la transformation de la sève selon la revendication 1, **caractérisé** en ce que la sève est hydrolysée en ajoutant une enzyme protéolytique telle que ALCALASE® .